# EUROPÄISCHE PATENTSCHRIFT

(11) **EP 0 479 881 B1**
(45) Veröffentlichungstag und Bekanntmachung des Hinweises auf die Patenterteilung: **08.02.1995**
(21) Anmeldenummer: 90910738.5
(22) Anmeldetag: 26.06.1990
(51) Int. Cl.: A61F 2/48, A61F 5/48

(54) **PROTHESE ZUR VERHINDERUNG DER HARNINKONTINENZ BEI FRAUEN**
PROSTHESIS FOR PREVENTING INCONTINENCE OF THE URINARY TRACT IN WOMEN
PROTHESE ANTI-INCONTINENCE POUR FEMMES

(30) Priorität: 28.06.1989 DE 3921120
(43) Veröffentlichungstag der Anmeldung: 15.04.1992
(73) Patentinhaber: RULL, Johann, Dr. med., 71522 Backnang (DE)
(72) Erfinder: RULL, Johann, Dr. med., 71522 Backnang (DE)
(86) Internationale Anmeldenummer: EP9001016
(87) Internationale Veröffentlichungsnummer: WO9100069

(56) Entgegenhaltungen:
- EP-A- 0 264 258
- GB-A- 2 189 999
- US-A- 3 066 667
- US-A- 3 789 828

## Beschreibung

Die Erfindung betrifft eine Prothese zur Verhinderung der Harninkontinenz bei Frauen, bei der ein vorgefertigter implantierbarer Formkörper als nach der Implantation veränderbarer Hohlkörper ausgebildet ist, der mit Nadeln durchstechbare und Nadelstiche selbst abdichtende Wandungsbezirke bzw. Wandungen aufweist.

Im Gegensatz zur männlichen Harnröhre besitzt die weibliche Harnröhre keinen eigentlichen Schließmuskel. Sie ist vielmehr, ausgehend von der Harnblase, mit Muskelfasern überzogen. Bei einer altersbedingten oder krankhaften Blasensenkung, die in erster Linie eine Senkung im Bereich des Harnröhrenansatzes ist, können diese Fasern nicht mehr dicht schließen, wenn eine Streßbeanspruchung erfolgt, zum Beispiel in Folge einer Druckbelastung beim Husten. Zur Verhinderung einer derartigen Streßinkontinenz ist es erforderlich, den Harnröhrenansatz wieder zu heben, um die natürliche Winkelstellung zwischen Harnröhre und Blase wieder herzustellen.

Hierzu gibt es sog. Zügelplastiken, bei denen man die Harnröhre im Bereich des Harnröhrenansatzes mit körpereigenen Faszien untergreift, welche in einem höher gelegenen Bereich des Bauchraumes befestigt werden. Derartige Zügelplastiken erfordern einen großen operativen Eingriff und sind bei einer falschen Bemessung der Faszienlänge bzw. bei nachträglichen Veränderungen der Lage der Blase nur schwer korrigierbar.

Die GB-A-21 89 999 beschreibt eine Inkontinenzprothese in Form eines halbmondförmigen Hydrogels mit einem Wassergehalt von ca. 90 Gew.-%. Zur Fixierung wird diese Prothese an benachbartes Gewebe angenäht. Auch hier sind Korrekturen nicht möglich oder mit großem Aufwand verbunden.

Es gibt auch sog. Ballonpressen, die um die Harnröhre herumgelegt werden und einen künstlichen Schließmuskel darstellen. Solche Pressen sind jedoch schwer handhabbar und behindern etwaige spätere Operationen an der Harnröhre selbst. Es wurde auch schon vorgeschlagen, Kunststoffmassen, wie sie für Körperplastiken verwendet werden, unter den Harnröhrenansatz zu spritzen. Auch diese Methode ist schwer beherrschbar.

Aus der US-A-3 789 828, die zur Formulierung des Oberbegriffs des Anspruchs 1 herangezogen worden ist, ist eine Prothese zur Verhinderung der Harninkontinenz bei Männern und Frauen bekannt, die als vorgefertigter implantierbarer Formkörper ausgebildet ist, die als Hohlkörper und nach der Implantation veränderbar ausgebildet ist und deren Hohlkörper mit Nadeln durchstechbare und Nadelstiche selbst abdichtende Wandungsbezirke bzw. Wandungen aufweist. Diese Prothese ist dazu ausgebildet, gegen die Harnröhre zu drücken und ihren Durchgang im Normalzustand zu verschließen und nur bei erhöhtem Harndruck zu öffnen. Die Probleme der Streßinkontinenz bei Frauen werden dabei nicht berücksichtigt.

Der Erfindung liegt die Aufgabe zugrunde, brauchbare Mittel zur Beseitigung der Streßinkontinenz zur Verfügung zu stellen, ohne daß große operative Eingriffe erforderlich sind.

Diese Aufgabe wird gelöst durch eine Prothese zur Verhinderung der Harninkontinenz bei Frauen, die dadurch gekennzeichnet ist, daß sie als Abstandhalter zwischen Vagina und Harnröhrenansatz ausgebildet ist und zumindest in ihrer den Abstand bestimmenden Größe durch Veränderung des Füllgrades des Hohlkörperhohlraumes mit Füllmedium einstellbar ist, wobei die Oberseite der Prothese eine zur Aufnahme der Harnröhre vorgesehene im wesentlichen U-förmige Rinne aufweist, die eine lichte Weite von mindestens etwa dem 1,5-fachen des Außendurchmessers der Harnröhre besitzt, und wobei die Wandung der U-förmigen Rinne eine größere Nachgiebigkeit besitzt als die übrigen Wandungsteile und die Unterseite der Prothese als Abstützfläche zur Auflage auf der Oberseite der Vagina ausgebildet ist.

Eine solche Prothese kann je nach dem Grad der Blasensenkung und der Größe der Patientin in ihrer Größe vorgefertigt werden und in ihrer Form den anatomischen Verhältnissen angepaßt sein. Für die Implantation ist kein großer operativer Eingriff erforderlich. Vielmehr kann sie durch einen kleinen Schnitt in der oberen Scheidewand in den Zwischenraum zwischen Harnröhrenansatz und Vagina eingesetzt werden, wozu im Normalfall eine örtliche Narkose ausreicht. Eine Öffnung des Bauchraumes von außen erübrigt sich. Gleichzeitig bleibt die Harnröhre zumindest von ihrer Oberseite her zugänglich, so daß eventuelle spätere Operationen nicht behindert werden. Auch eine eventuelle spätere Entfernung des Abstandhalters bzw. dessen Austausch ist in einfacher Weise möglich.

Die Prothese nach der Erfindung ist als verstellbarer Abstandhalter ausgebildet. Hierzu ist die Prothese zumindest in ihrer den Abstand des Abstandhalters bestimmenden Größe einstellbar und insbesondere auch nach der Implantation veränderbar ausgebildet. Dabei kann die Prothese so ausgebildet sein, daß sie in ihrer gesamten Größe veränderbar ist. In der Regel reicht jedoch eine Veränderbarkeit der Größe der den Abstandhalter bildenden Bereiche aus, wobei die anderen Teile im wesentlichen in der Größe unverändert bleiben können. Vorzugsweise ist die Prothese mindestens zum Teil aus gummielastischem Material gefertigt, das insbesondere weichelastisch und mindestens teilweise nachgiebig ist. Dadurch wird die Prothese nicht als störender Fremdkörper empfunden. Außerdem wird dadurch die Größenverstellbarkeit erleichtert. Der Hohlraum ist vorzugsweise mit einer Flüssigkeit, beispielsweise isotonischer Kochsalzlösung, gefüllt und zwar vorzugsweise vollständig gefüllt. Es ist aber auch eine Befüllung mit einem Gas, insbesondere einem Inertgas möglich.

Zur Einstellung bzw. zur Veränderung des Abstandes des Abstandhalters ist das Volumen des Hohlkörpers veränderbar ausgebildet, vorzugsweise gegen einen geringen Gegendruck. Dieser kann mit Vorteil etwas über dem im Bauchraum herrschenden Druck liegen. Ermöglicht wird die Veränderbarkeit im Volumen durch elastische Wandungen des Hohlkörpers. Dabei ist die Elastizität des Wandungsmaterials und/oder die Wandungsstärke an verschiedenen Stellen des Hohlkörpers verschieden, so daß sich die Größenänderungen bei Volumenveränderung an unterschiedlichen Stellen der Prothese verschieden auswirken. So ist die Wandung des Hohlkörpers mit Vorteil an den den Abstand des Abstandhalters bestimmenden Wandungsteilen weichelastischer bzw. nachgiebiger als an anderen Stellen, so daß sich Volumenänderungen in einer Veränderung des Abstandes auswirken. Dabei besitzt die Prothese insbesondere im Bereich der Harnröhre zen mit Vorteil eine höhere Weichelastizität, so daß sich Volumenänderungen dort auswirken, worauf später noch näher eingegangen wird.

Der Formkörper der Prothese ist vorzugsweise so ausgebildet, daß er nicht nur als Abstandhalter zwischen Harnröhre und Vagina dient, sondern durch die Abstützung an diesen auch in seiner Lage festgehalten wird. Dadurch sind besondere Befestigungen, zum Beispiel eine Fixierung an bestimmten Organen oder Gewebeteilen, nicht erforderlich. Dazu ist die Prothese als Profil ausgebildet, dessen Querschnitt über seine Länge im wesentlichen konstant ist. Zu seiner Fixierung und zur Aufnahme der Harnröhre weist die Prothese an ihrer Oberseite eine im wesentlichen U-förmige Rinne auf, deren Innendurchmesser mindestens um etwa das 1,5-fache größer ist als der Außendurchmesser der Harnröhre, so daß diese im Normalzustand nicht eingeengt wird. Die Basis der U-förmigen Rinne, d.h. die eigentliche Abstützfläche für die Harnröhre, ist vorzugsweise abgeflacht. Die Längsstege der U-förmigen Rinne können nach oben so weit verlängert sein, daß sie die Harnröhre zu zwei Drittel ihres Umfanges oder mehr umfassen. Wesentlich ist jedoch, daß die Längsränder der Rinne bzw. die nach oben ragenden Stege des entsprechenden Profilquerschnittes an ihren Enden nicht miteinander verbunden sind und insbesondere dann elastisch aufweitbar sind, wenn sie im oberen Bereich gegeneinander gerichtet sind. Dadurch bleibt die Harnröhre für spätere Eingriffe von oben her zugänglich. Weiterhin kann die Harnröhre leicht in die U-förmige Rinne eingelegt werden.

Die Längsränder der U-förmigen Rinne sind vorzugsweise ebenfalls Teil des Hohlkörpers. Dadurch besitzen sie eine gute Elastizität. Außerdem ist es möglich, ihre Lage und Dicke durch Veränderung des Volumens des Hohlkörpers zu verändern. Vorzugsweise sind die Innenwandungen der Längsränder für Druckänderungen nachgiebiger als andere Wandungsteile der Längsränder. Durch Pressung auf den Hohlkörper ist der lichte Abstand zwischen den Längsrändern somit verkleinerbar, wodurch eine Pressung auf die Harnröhre und damit eine Erhöhung ihrer Schließkraft bewirkt werden kann, was bei Streßsituationen, zum Beispiel beim Husten, hilfreich ist.

Die Volumenänderung des Hohlraumes des Hohlkörpers ist erfindungsgemäß durch eine Veränderung des Füllgrades des elastischen Hohlkörpers durchführbar. Hierzu kann der Hohlkörper ein Ventil zum Einführen oder Ablassen von Füllmedium aufweisen. Erfindungsgemäß besteht die Wandung des Hohlkörpers an mindestens einem zugänglichen Bereich, vorzugsweise insgesamt, aus einem Material, das mit Spritzennadeln durchstechbar ist und sich nach Herausziehen der Nadel von selbst abdichtet. Derartige Materialien sind in der Medizin bekannt. Durch Einstechen einer Nadel und durch Zuführen oder Abziehen von Füllmedium kann somit das Volumen und insbesondere der von der Prothese zu schaffende Abstand eingestellt bzw. verändert werden. Aufgrund dieser Ausbildung ist dies auch noch nach der Implantation möglich, da die Prothese leicht durch einen Stich durch die Wand der Vagina von der Unterseite her zugänglich ist. Die Prothese weist deshalb vorzugsweise an ihrer Unterseite Wandbereiche aus dem selbst abdichtenden Material auf.

Um etwaige Diffusionen und sonstige Veränderungen zu vermeiden, kann der Hohlkörper auch mehrschichtig aus verschiedenartigen Materialien ausgebildet sein, so zum Beispiel aus einer Kombination von Siliconkautschuk und/oder Naturkautschuk und/oder anderen synthetischen Kautschuken, um einerseits die gewünschte Festigkeit und andererseits Dichtigkeit und insbesondere Abdichtbarkeit zu erreichen.

Die Unterseite der Prothese ist zur flächenhaften Auflage auf der Oberseite der Vagina ausgebildet. Hierzu kann die Unterseite im wesentlichen eben ausgebildet sein. Mit Vorteil ist sie als konkave Auskehlung ausgebildet, deren Längsrichtung im wesentlichen parallel zur U-förmigen Rinne der Oberseite verläuft. Die durch die Auskehlung gebildete Rinne ist vorzugsweise weiter und flacher als die U-förmige Rinne an der Oberseite. Die Längsränder der Prothese an der Unterseite sind vorzugsweise ebenfalls weichelastisch. Mit Vorteil sind sie als Teil des Hohlkörpers ausgebildet, vorzugsweise doppelwandig.

Die Größe der Prothese richtet sich nach den vorgegebenen anatomischen Verhältnissen. Sie kann je nach der Körpergröße der Patientin und der erforderlichen Anhebung des Harnröhrenansatzes in verschiedenen Grundgrößen vorgefertigt sein, wobei die Zwischengrößen dann durch Veränderung des Abstandes erreicht werden können. Normalerweise reicht eine Länge parallel zur Längsrichtung der Harnröhre von 15 bis 40 mm, insbesondere ca. 20 mm, aus. Die Höhe des Abstandhalters, d.h. der Abstand zwischen der unteren Auflagefläche zur Auflage auf der Oberseite der Vagina und der oberen Abstützfläche zur Aufnahme der Harnröhre liegt normalerweise im Bereich zwischen 15 und 30 mm, insbesondere ca. 20 mm und ist variierbar. Die Gesamtbreite und Gesamthöhe der Prothese liegt vorzugsweise im Bereich zwischen 25 und 50 mm, insbesondere 30 bis 40 mm.

Die der Blase zugewandte Stirnseite der Prothese ist vorzugsweise abgeschrägt, insbesondere konkav ausgebildet. Dadurch wird erreicht, daß die Prothese mit dieser Stirnseite die Blase im Bereich des Harnröhrenansatzes teilweise untergreift, wodurch eine bessere Unterstützung des Harnröhrenansatzes und eine gute Fixierung der Prothese erreicht werden.

Bei bevorzugten Ausführungsformen der Erfindung weist die Prothese einen Profilquerschnitt auf, der im wesentlichen dem eines H entspricht, wobei die unteren Stege des H vorzugsweise seitlich mehr oder weniger stark nach außen abgebogen sind. In einfachen Fällen kann die Prothese im Querschnitt auch U-förmig ausgebildet sein, wobei der Quersteg bzw. die Basis des U an der Unterseite eben oder konkav ausgebildet sein kann. In allen Fällen bildet die Höhe des Quersteges den eigentlichen Abstandhalter zur Anhebung des Harnröhrenansatzes und zur Herstellung des natürlichen Winkels zwischen Harnröhre und Blase.

Weitere Merkmale, Einzelheiten und Vorteile der Erfindung ergeben sich aus der nachfolgenden Beschreibung von bevorzugten Ausführungsformen in Verbindung mit der Zeichnung und den Unteransprüchen. In der Zeichnung zeigen:
- Fig. 1: einen Querschnitt durch eine bevorzugte Ausführungsform nach der Erfindung, wobei der implantierte Zustand schematisch dargestellt ist,
- Fig. 2: eine Seitenansicht der Ausführungsform nach Fig. 1 und
- Fig. 3: einen Querschnitt durch eine andere Ausführungsform.

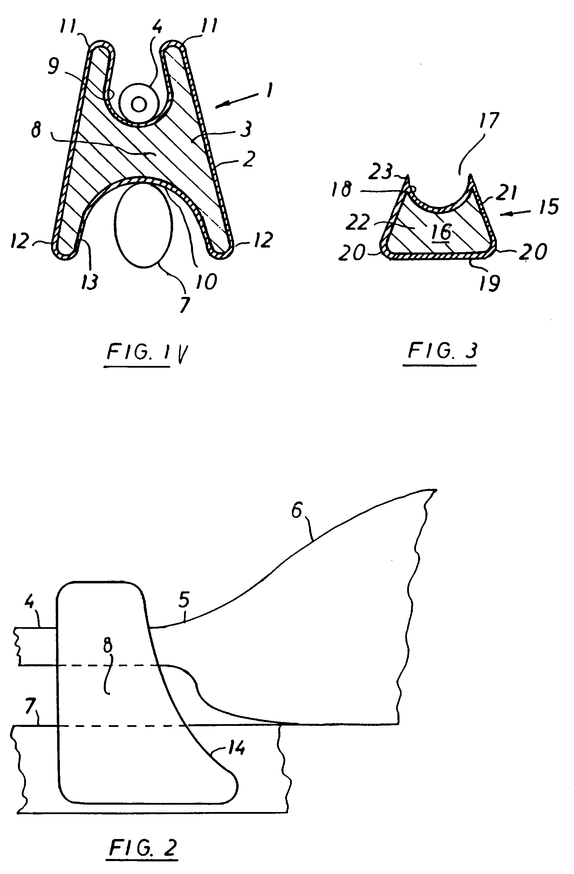

Bei der in der Zeichnung in den Fig. 1 und 2 dargestellten Ausführungsform der Erfindung weist eine Harninkontinenz-Prothese 1 einen Hohlkörper 2 aus weichelastomerem Material auf, der zum Beispiel durch Hohlraumspritzen oder -blasen gefertigt ist. Der Hohlkörper 2 ist als im Querschnitt etwa H-förmiges Profil ausgebildet, das eine Länge von ca. 20 mm besitzt. Der Hohlraum 3 des Hohlkörpers 2 ist mit physiologischer Kochsalzlösung gefüllt. Es sind auch andere physiologisch verträgliche Fluide, insbesondere Flüssigkeiten, möglich. Die Prothese 1 ist operativ zwischen die Harnröhre 4 im Bereich des Harnröhrenansatzes 5 an der Harnblase 6 und die Vagina 7 einsetzbar und dient dazu, bei einer altersbedingten oder krankhaften Senkung des Harnröhrenansatzes 5 den anatomisch richtigen Austrittswinkel des Harnröhrenansatzes durch dessen Anhebung wieder herzustellen.

Der Quersteg 8 des H-förmigen Profiles bildet dabei einen Abstandhalter, der im implantierten Zustand mit seiner Unterseite auf der Oberseite der Vagina 7 aufliegt und mit seiner Oberseite die Harnröhre 4 im Bereich des Harnröhrenansatzes 5 trägt. Der Quersteg 8 ist doppelwandig ausgebildet. Der Abstand zwischen der oberen Wand 9 und der unteren Wand 10 ist in Abhängigkeit vom Füllvolumen des Hohlkörpers 2 einstellbar bzw. veränderbar. Die nach oben ragenden Stege 11 des H-Profiles bilden zusammen mit dem Quersteg 8 eine in der Basis vorzugsweise abgeflachte, im wesentlichen U-förmige Rinne, die zur Aufnahme der Harnröhre 4 dient und gleichzeitig die Prothese 1 in ihrer Gebrauchslage fixiert. Die nach oben ragenden Stege 11 sind an ihrer Innenseite zusammen mit der oberen Wandung 9 des Quersteges 8 im Querschnitt im wesentlichen kreisbogenförmig ausgebildet und bilden einen Kreisbogen von etwa 240°, wobei der lichte Innendurchmesser des Kreisbogens etwa das 1,5-fache des Außendurchmessers der Harnröhre 4 beträgt. Er kann auch größer sein, vorzugsweise das 1,5- bis 3-fache betragen. Die nach oben ragenden Stege 11 des H-Profils sind ebenfalls doppelwandig ausgebildet und Teil des Hohlkörpers 2. Durch Pressung auf die Prothese 1, zum Beispiel beim Husten, blähen sich die Stege 11 auf, wodurch der lichte Abstand zwischen ihnen verringert wird und eine die Harnröhre 4 einschließende Verengung zustande kommt. Dadurch wird die Schließkraft der Harnröhre 4 gegen ungewollten Harnabgang erhöht. Diese Verengung des lichten Abstandes ist bei der Ausführungsform dadurch begünstigt, daß die Innenwand der U-förmigen Rinne etwas nachgiebiger ist als die Außenwand des Hohlkörpers 2.

Die nach unten ragenden Stege 12 des H-Profiles bilden mit der unteren Wand 10 des Quersteges 8 ebenfalls eine im wesentlichen U-förmige Rinne bzw. Kehlung, die jedoch flacher ausgebildet ist als die obere. Die untere Rinne bzw. Kehlung dient zur Abstützung der Prothese 1 auf der Oberseite der Vagina 7, wobei die Stege 12 seitlich nach außen geneigt sind und eine im wesentlichen flächenhafte Auflage und eine Stabilisierung gegen seitliches Verrutschen bieten. Die nach unten ragenden Stege 12 sind ebenfalls weichelastisch. Sie sind bei der dargestellten Ausführungsform ebenfalls doppelwandig ausgebildet und sind Teil des Hohlkörpers 2.

Die Wandung der Prothese besteht zumindest im Bereich der zur Auflage auf der Vagina bestimmten Unterseite 13 aus punktionsfähigem Material, das Einstiche von Spritzennadeln ermöglicht und sich nach Herausziehen der Nadel selbst abdichtet. Hierdurch ist eine Größeneinstellung des Abstandes zwischen der oberen Wandung 9 und der unteren Wandung 10 des Quersteges 8 vor der Implantation möglich. Diese Ausführung erlaubt auch spätere Korrekturen dieses Abstandes, ohne daß die Prothese operativ entfernt zu werden braucht, da der Hohlraum 3 der Prothese durch die obere Scheidenwand und durch die Unterseite der Prothese mit Hilfe einer Spritzennadel zugänglich ist. Die obere Wand 9 des Quersteges 8 ist ebenfalls von größerer Nachgiebigkeit als die übrigen Wandungsteile der Prothese und vorzugsweise auch der seitlichen Innenwände der Stege 11. Dadurch wird erreicht, daß sich Volumenänderungen und insbesondere Druckerhöhungen in erster Linie im Bereich des Abstandhalters auswirken. Bei stärkeren Pressungen kommt dann noch eine Verengung der oberen U-förmigen Rinne hinzu.

Die erfindungsgemäße Prothese kann in einfacher Weise in die Gebrauchslage gebracht werden. Hierzu reicht ein kurzer Schnitt an der oberen Wandung der Vagina aus, um die Prothese durch diese hindurch einführen und in ihre Lage bringen zu können. Die blasenseitige Stirnseite 14 der Prothese ist vorzugsweise von oben nach unten abgeschrägt, so daß die Prothese teilweise die Blase im Bereich des Harnröhrenansatzes 5 untergreift.

Die Größe der Prothese ist den anatomischen Verhältnissen angepaßt. Insgesamt ist die Prothese in Längsrichtung des Profiles kürzer als in der Höhe und in der Breite, da es in erster Linie auf eine Abstützung des Harnröhrenansatzes 5 ankommt. Bei Bedarf kann die Prothese in Längsrichtung des Profiles auch länger gehalten werden.

In Fig. 3 ist eine einfachere Ausführungsform der Erfindung dargestellt. Hier hat die Prothese 15 etwa die Form eines Profiles 16 mit trapezförmigen Außenlinien, wobei die Oberseite 17 zur Ausbildung der U-förmigen Rinne 18 in Längsrichtung flach ausgekehlt ist. Die Unterseite 19 der Prothese 15 ist im wesentlichen eben, vorzugsweise leicht konkav ausgebildet. Die Längsränder 20 der Unterseite 19 bestehen wie der übrige Körper der Prothese wieder aus weichelastischem Material, zum Beispiel Siliconkautschuk, die Wandung 21 des Hohlkörpers 22 der Prothese ist jedoch im Gegensatz zu den Wandungen der Ausführungsform nach den Fig. 1 und 2 nicht von im wesentlichen gleichbleibender Dicke. Vielmehr sind hier stärkere Unterschiede vorgesehen, wobei die größte Nachgiebigkeit jedoch wiederum an der Innenwandung 23 der oberen U-förmigen Rinne vorgesehen ist.

Die Erfindung ist nicht auf die beschriebene Ausführungsform beschränkt. Es sind vielmehr Abwandlungen und Änderungen möglich, ohne daß der Rahmen der Erfindung verlassen wird. So kann die erfindungsgemäße Prothese in drei verschiedenen Größen vorgefertigt und vorrätig sein. Durch entsprechende Vergrößerung infolge einer Vergrößerung des Füllgrades des Hohlraumes lassen sich alle gewünschten Zwischengrößen für den Abstandhalter erreichen, die je nach den anatomischen Bedürfnissen und der Körpergröße der Patientin erwünscht sind. Wie aus den Fig. 1 und 3 ersichtlich, steht für ein Punktieren der Prothese von der Unterseite her genügend Raum für die Einstichtiefe der Punktierungsnadel zur Verfügung. Dadurch wird vermieden, daß die Seitenwand oder die obere Wand der Prothese versehentlich mit durchstochen wird. Eine zusätzliche Kontrolle bei der Punktierung kann dadurch geschaffen werden, daß die Flüssigkeit im Hohlkörper gefärbt ist. Durch Zurückziehen des Spritzenkolbens kann dann anhand der Farbe der in die Spritze eintretenden Flüssigkeit kontrolliert bzw. sichergestellt werden, daß sich die Spitze der Spritze der Nadel an der richtigen Stelle befindet. Die Punktierungsstellen für den Hohlkörper sind vorzugsweise im Bereich von ebenen und vorzugsweise konkaven, nicht aber im Bereich von konvexen Außenflächen der Prothese vorgesehen. Dadurch wird die Dichtigkeit der Prothesen auch nach mehrmaliger Punktion begünstigt, weil diese Wandungsstellen bei einer Druckerhöhung innerhalb der Prothese nicht oder nur wenig geweitet werden. Die Wandungsstärke der Prothese kann je nach Festigkeit und Elastizität des gummielastischen Materials im Bereich von 0,5 bis 5 mm liegen, wobei 1 bis 2 mm in der Regel ausreichen. Im Bereich der Punktierungsstellen können Verstärkungen der Wandung vorgesehen sein.

Wie oben bereits erwähnt, ist eine besondere Befestigung der Prothese im Körper nicht erforderlich, da sie sich aufgrund ihrer Form, Größe und Abstandhalter-Funktion zwischen der Oberseite der Vagina und der Unterseite der Harnröhre selbst abstützt. Gleichzeitig können die oberen seitlichen Ränder der Prothese, beispielsweise die Stege 11 der Ausführungsform nach Fig. 1, die absteigenden Äste des Schambeins hintergreifen, d.h. mit ihrer Vorderseite im wesentlichen an der Rückseite der Schambeinäste anliegen, so daß die Prothese auch in ihrer axialen Richtung zwischen Blase und Schambein fixiert ist. Das Hintergreifen kann von anatomischen Größenverhältnissen weitgehend unabhängig erreicht werden, indem die oberen Ränder der Prothese seitlich nach außen erweitert ausgebildet sind bzw. verlaufen.

## Patentansprüche

1. Prothese zur Verhinderung der Harninkontinenz bei Frauen, bei der ein vorgefertigter implantierbarer Formkörper (2; 22) als nach der Implantation veränderbarer Hohlkörper ausgebildet ist, der mit Nadeln durchstechbare und Nadelstiche selbst abdichtende Wandungsbezirke bzw. Wandungen (10; 19) aufweist, dadurch gekennzeichnet, daß sie als Abstandhalter zwischen Vagina (7) und Harnröhrenansatz (5) ausgebildet ist und zumindest in ihrer den Abstand bestimmenden Größe (9, 10) durch Veränderung des Füllgrades des Hohlkörperhohlraums mit Füllmedium einstellbar ist, wobei die Oberseite der Prothese eine zur Aufnahme der Harnröhre (4) vorgesehene, im wesentlichen U-förmige Rinne (9; 18) aufweist, die eine lichte Weite von mindestens etwa dem 1,5-fachen des Außendurchmessers der Harnröhre (4) besitzt, wobei die Wandung (9, 23) der U-förmigen Rinne eine größere Nachgiebigkeit besitzt als die übrigen Wandungsteile, und die Unterseite (10; 19) der Prothese als Abstützfläche zur Auflage auf der Oberseite der Vagina (7) ausgebildet ist.

2. Prothese nach Anspruch 1, dadurch gekennzeichnet, daß der Hohlkörper mit gummielastischen Wandungen (9, 10) ausgebildet ist.

3. Prothese nach Anspruch 1 oder 2, dadurch gekennzeichnet, daß sie aus gummielastischem Material gefertigt ist.

4. Prothese nach einem der Ansprüche 1 bis 3, dadurch gekennzeichnet, daß das Füllmedium Flüssigkeit ist.

5. Prothese nach einem der Ansprüche 2 bis 4, dadurch gekennzeichnet, daß die Wandung (9, 10; 21) des Hohlkörpers (2; 22) mehrschichtig, insbesondere aus verschiedenen gummielastischen Materialien, ausgebildet ist.

6. Prothese nach einem der vorhergehenden Ansprüche, dadurch gekennzeichnet, daß ihre Unterseite (10) als konkave Rinne ausgebildet ist, die im wesentlichen Spiegelbildlich zur U-förmigen Rinne (9, 11) der Oberseite verläuft und vorzugsweise flacher ausgebildet ist als diese.

7. Prothese nach einem der vorhergehenden Ansprüche, dadurch gekennzeichnet, daß die Unterseite (10; 19), insbesondere die konkave Rinne, elastisch rückfedernde Längsstege (12; 20) aufweist.

8. Prothese nach einem der vorhergehenden Ansprüche, dadurch gekennzeichnet, daß ihre blasenseitige Stirnseite (14) zum mindestens teilweisen Untergreifen der Harnblase (6) im wesentlichen konkav ausgebildet ist.

## Claims

1. A prosthesis for preventing urinary incontinence in females, wherein a prefabricated implantable shaped article (2,22) is constructed as a hollow body modifiable after implantation, has wall areas or walls (10,19) which can be perforated with needles and in which the needle perforations self-seal, wherein it is constructed as a spacer between the vagina (7) and the urethral attachment (5) and wherein at least its size (9,10) determining the spacing is adjustable through changes to the filled level of the cavity of the hollow body with filling medium, wherein the top of the prosthesis has a substantially U-shaped channel (9,18) for receiving the urethra (4), which has an internal diameter of at least approximately 1.5 times the external diameter of the urethra (4), wherein the walls (9,23) of the U-shaped channel exhibit a greater elasticity than the other wall parts, wherein the bottom (10,19) of the prosthesis is constructed as a support surface for bearing on top of the vagina (7).

2. A prosthesis according to claim 1, wherein the the walls (9,10) of the hollow body are made from rubber elastic material.

3. A prosthesis according to claim 1 or 2, wherein it is made from rubber elastic material.

4. A prosthesis according to claims 1 to 3, wherein the filling medium is liquid.

5. A prosthesis according to claims 2 to 4, wherein the wall (9,10,21) of the hollow body (2,22) is constructed in multilayer form, particularly from different rubber elastic materials.

6. A prosthesis according to one of the preceding claims wherein the bottom (10) is constructed as a concave channel, which is substantially homologous to the U-shaped channel (9,11) of the top and is preferably shallower than the latter.

7. A prosthesis according to one of the preceding claims, wherein the bottom (10,19), particularly the concave channel, has elastic, resilient longitudinal webs (12,20).

8. A prosthesis according to one of the preceding claims, wherein the bladder-side face (14) is made substantially concave for the at least partial engagement below the bladder (6).

## Revendications

1. Prothèse de prévention de l'incontinence urinaire féminine, consistant en un élément moulé préfabriqué implantable (2; 22) conçu en corps creux variable après implantation, présentant des secteurs de parois ou des parois (10; 19) perforables avec des aiguilles et auto-étanchéifiantes par des piqûres d'aiguilles, caractérisée par le fait qu'elle est conçue comme écarteur entre le vagin (7) et le col de la vessie (5) et étant au moins variable dans sa grandeur (9, 10), déterminante de l'écart, par modification du degré de remplissage de la cavité du corps creux par un agent, la partie supérieure de la prothèse présentant un canal, pour la plus grande partie en forme de U (9, 18), prévu pour recevoir l' urètre (4), ayant une largeur intérieure supérieure d'au moins 1,5 fois au diamètre extérieur de l'urètre (4), la paroi (9, 23) du canal en forme de U ayant une élasticité supérieure à celle des autres parties de la paroi, la partie inférieure (10, 19) de la prothèse étant conçue comme face d'appui reposant sur la partie supérieure du vagin (7).

2. Prothèse selon Revendication 1, caractérisée par le fait, que le corps creux est formé de parois d'une élasticité de caoutchouc (9, 10).

3. Prothèse selon Revendication 1 ou 2, caractérisée par le fait, qu'elle est conçue en matériel d'une élasticité de caoutchouc.

4. Prothèse selon Revendications 1 à 3, caractérisée par le fait, que l'agent de remplissage est du liquide.

5. Prothèse selon Revendications 2 - 4, caractérisée par le fait, que la paroi (9, 10, 21) du corps creux (2; 22) est formée de plusieurs couches, en particulier de divers matériaux d'une élasticité de caoutchouc.

6. Prothèse selon l'une des Revendications ci-dessus, caractérisée par le fait, que sa partie inférieure (10) est conçue en canal concave, qui, pour l'essentiel, représente le tracé réfléchi du canal en forme de U (9, 11 ) de la partie supérieure et est de préférence plus plat que celui-ci.

7. Prothèse selon l'une des Revendications ci-dessus, caractérisée par le fait, que sa partie inférieure (10, 19), en particulier le canal concave, présente des traverses longitudinales à retour élastique (12; 20).

8. Prothèse selon l'une des Revendications ci-dessus, caractérisée par le fait, que sa partie frontale du côté de la vessie (14), est pour l'essentiel concave, ce pour glisser au moins en partie sous la vessie (6).
